(19) Europäisches Patentamt
European Patent Office
Office européen des brevets

(11) EP 1 373 238 B1

(12) EUROPEAN PATENT SPECIFICATION

(45) Date of publication and mention of the grant of the patent:
**03.11.2004 Bulletin 2004/45**

(21) Application number: **01932049.8**

(22) Date of filing: **30.03.2001**

(51) Int Cl.[7]: **C07D 307/12**, C07C 329/16, A01N 47/06, A01N 43/08

(86) International application number:
**PCT/IN2001/000081**

(87) International publication number:
**WO 2002/079179 (10.10.2002 Gazette 2002/41)**

# (54) ALKYLXANTHATES AND THEIR USE AS PESTICIDES

ALKYLXANTHATE UND IHRE ANWENDUNG ALS SCHÄDLINGSBEKÄMPFUNGSMITTEL

ALKYLXANTHATES ET LEUR UTILISATION COMME PESTICIDES

(84) Designated Contracting States:
**AT BE CH CY DE DK ES FI FR GB GR IE IT LI LU MC NL PT SE TR**

(43) Date of publication of application:
**02.01.2004 Bulletin 2004/01**

(73) Proprietor: **Council of Scientific and Industrial Research;an Indian Reg. body incorporated under Reg. of Societies Act (Act XXI of 1860)
New Delhi 110 001 (IN)**

(72) Inventors:
- **RAO, Janapala, Venkateswara
Hyderabad, Andhra Pradesh 500 007 (IN)**
- **VENKATESWARLU, Yenamandra
Hyderabad, Andhra Pradesh 500 007 (IN)**
- **RAGHAVAN, Kondapuram, Vijaya
Andhra Pradesh 500 007 (IN)**

(74) Representative: **Appelt, Christian W.
FORRESTER & BOEHMERT
Anwaltssozietät
Pettenkoferstrasse 20-22
80336 München (DE)**

(56) References cited:
**US-A- 2 011 302** **US-A- 3 011 887**

- **J. VENKATESWARA RAO ET AL: INDIAN JOURNAL OF EXPERIMENTAL BIOLOGY, vol. 33, 1995, pages 399-400, XP000926514**
- **S.V. ZHURAVLEV : ZH. PRIKL. KHIM. (ENGL. ED.), vol. 23, 1950, pages 1159-1163, XP000926517 LENINGRAD**
- **S. WAKAMORI ET AL: AGR. BIOL. CHEM. , vol. 33, no. 12, 1969, pages 1682-1690, XP000926516**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 373 238 B1

## Description

### Technical Filed

The present invention relates to novel alkylxanthates of formulae 1 to 3 and use of alkylxanthates of formulae 1, 2, 3, and 5 in the integrated pest management, more specifically alkyl xanthates are used as insect growth regulators for pest management in agricultural fields with no or less environmental impact.

### Background Art

Many small scale farmers are illiterate and believe in the usage of high volumes, high pressure, and high doses of pesticide as the most suitable way of crop protection, these practices have resulted in many environmental problems such as effect on non-target species, pesticide residue accumulation, variable selectivity, development of resistant in pests, ecological imbalance by elimination of natural predators as well as and environment pollution. More than 50% of the pesticides used in third world countries are globally banned and 95% of the used by the public health sector also find restricted use in other nations (News corner, Pesticide World, 1998). There are 33 pesticides, which have been banned in advanced countries, but they are allowed to be imported and used in third world countries (Insecticides Act, 1968 and Indian Express Newspaper January, 2000). In order to restrict the use of banned pesticides, they must be replaced with suitable pesticides that must be target specific, non-toxic to other species, easily biodegradable and less persistent.

Hence, the advanced countries are mainly concentrated to identify the IV generation of insecticides, which are highly effective and generally selective agents derived for tactical skills. It requires more management expertise which are far friendlier to the total environment including the biological control organisms.

The applicants have isolated a Ethylene Bis (isobutylxanthate) (4) from a green alga *Dictyosphaeria-favulosa* (Venkateswarlu *et al., 1993).* It was tested against various aquatic stages of *Aedes aegypti* mosquito, which is a yellow fever vector. The compound 4 exhibited larvicidal and insect growth regulatory activity in dose dependent manner (Venkateswara Rao *et al*., *1995*)*.* Around thirty-five analogues were synthesised and tested against agricultural polyphagous lepidopteron pests. Among them four analogues have been identified as good antifeedant and insect growth regulators. Two of the compounds from the four analogues are totally new structures and remaining two are structurally known but the activity was identified for the first time.

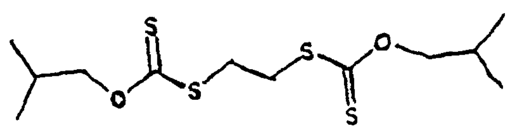

4

| | |
|---|---|
| Compound 4 : | Ethylene Bis (isobutylxanthate) |
| Physical state : | Colorless solid |
| M. P : | 40-42°C |
| Molecular formula : | $C_{12}H_{22}O_2S_4$ |
| EIMS (70 eV) : | *m/z* 326 |
| Elemental analysis : | Found: C 44.15 %, H 6.90 %, S 39.15 % |
| | Required: C 44.13 %, H 6.79 %, S 39.28 % |
| IR (KBr) : | $\nu_{max}$ (KBr) 2950, 1465, 1235, 1200, and 1140 $cm^{-1}$ |
| UV (MeOH) : | $\lambda_{max}$ (MeOH) 217 (ε 12725),232 (14766) and 278 nm (20048) |
| $^1$H NMR : | ($CDCl_3$, 200 MHz) δ1.0 (6H, d, *J* = 7.5 Hz), 2.15 (1H, m), 3.42 |

In 1950 S. V. Zhuravlev, reported the synthesis of ethylene dixanthates (2'-5'). Heating the mixture of 4.7 gms $(CH_2Br)_2$ and $EtOCS_2K$ in ethanol on a steam bath gave ethylene . dixanthate (yield 90 %). In which compounds 2' and 5' possess a moderate insecticidal activity against lice.

In 1954 Yamasaki, reported the synthesis of organophophorus compounds (xanthogenates). A series of xanthogenates (6'-10') were synthesized in three ways.

A) 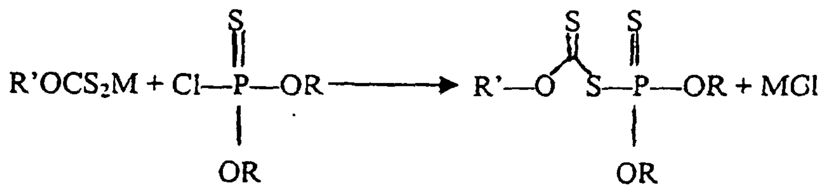

B) 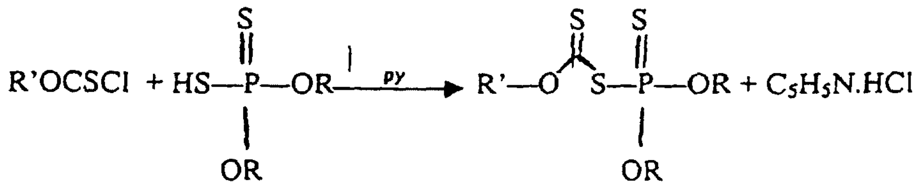


C) 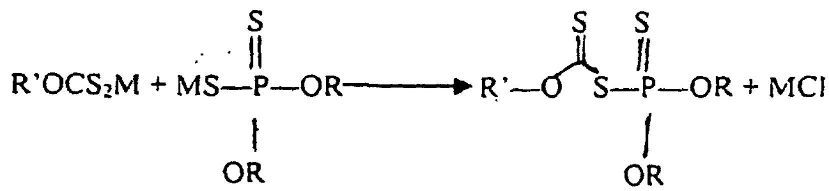

here M=alkali metal
6' R'=Me; R=Me
7' R'=n-propyl; R=ethyl
8' R'=ethyl; R=ethyl
9' R'=isopropyl; R=ethyl
10' R'=n-butyl; R=ethyl

In 1950 S. V. Zhuravlev, reported the synthesis of p-xylene dixanthates. $MeOCS_2K$ reacted with p-$C_6H_4(CH_2Cl)_2$ in ethanol gave p-xylene bis (methytxanthate) (yield 75.5 %). A series of p-xylene bis xanthates has been synthesised (11-20). All these bis xanthates are insecticides with low order of activity.

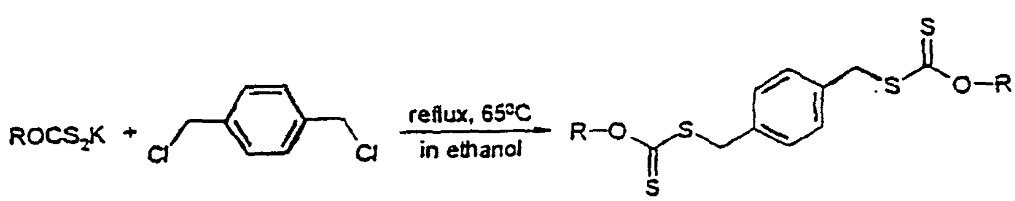


11 R=ethyl
12 R=n-butyl (Figure-6)
13 R=isobutyl
14 R=octyl
15 R=isooctyl
16 R=nonyl
17 R=decyl
18 R=cyclohexyl
19 R=phenethyl
20 R=benzyl

The dixanthates were dissolved in refined kerosene to form a 25 % solution was used as a efficient defoliants for cotton plants (Cupery, 1958).

Etylene Bis (isopropylxanthate) (21) and ethylene bis(isobutylxanthate) (4) were used as noncorrosive antiseize lubricating oil additives (Khalikov and Vinogradova, 1964).

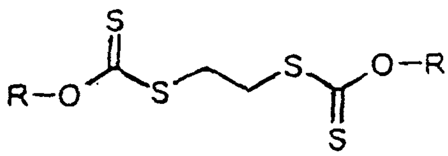

4 R=isobutyl
21 R=isopropyl

Methylene bisxanthates are used as collector reagents in the froth flotation of sulfide minerals (Saphores, *et al*., 1991).

Ten newly synthesized organo phosphorus derivatives containing alkyl xanthates were tested for their antifungal activity against *Colletotrichum falcatum, Fusarium oxisporium, Curvularia pallescens* (sugarcane pathogens). The O,O-diethyl thio phosphate derivatives exhibited 100 % mycelial inhibition against all the test fungi at 1000 ppm. All thio phosphate derivatives showed fungistatic effects at a minimum inhibitory concentration of 1000 ppm. These derivatives also proved better than currently used synthetic fungicides (Senguptha *et al*., 1998).

In the course, the applicants have synthesized ethylene bis (isobutylxanthate) and its analogues. In which compound 1, 2 and 3 are newly synthesized compounds and these compounds were tested for antifeedant and larvicidal activities.

**Objects of the invention**

The main object of the invention is to provide novel alkylxanthates for agricultural applications.

In another object of the invention is to provide a new use of novel alkylxanthates for use in integrated pest management.

In still another object is to provide a new activity of alkylxanthates for use in integrated pest management.

In yet another object is to provide a process for the preparation analogues of alkyl xanthates useful as an antifeedent and as insect growth regulators.

In yet another object is to provide a composition useful as an insect growth regulator and antifeedant for the integrated pest management.

**Summary of the invention**

The present invention relates to novel alkylxanthates of formulae 1 to 3 and use of alkylxanthates of formulae 1, 2, 3 and 5 in the integrated pest management, more specifically alkyl xanthates are used as insect growth regulators for pest management in agricultural fields with no or less environmental impact.

**Brief description of the accompanying drawings**

**Figure-1** shows structure of Methylene bis (tetrahydrofurfurylxanthate.
**Figure-2** shows structure of m-Fluorobenzyl n-butylxanthate
**Figure-3** shows structure m-Fluorobenzyl isobutylxanthate
**Figure-4** shows structure Ethylene bis (isobutylxanthate
**Figure-5** shows structure of Methylene bis (n-butylxanthate)
**Figure-6** shows structure p-Xylene bis (n-butylxanthate)
**Figure-7** shows glimpse of results wherein,

- 7a Control pupae and normal adult formation in polyphagous pest *Spodoptera litura*
- 7b Control Larvae
- 7c Xanthate treated larvae (Topical application)

7d effect on Larval Mortality
7e effect of Methylene bis (n-butylxanthate) 25μg/insect
7f effect of Methylene bis (tetrahydrofurfurylxanthate) 30μg/insect
7g effect of m-Fluorobenzyl n-butylxanthate 35μg/insect and

**Figure-8** shows glimpses of the another set of results wherein,

Fig 8a- Methylene bis (tetrahydrofurfurylxanthate) 20μg/insect
Fig 8b- p-Xylene bis (n-butylxanthate) (30μg/insect)
Fig 8c- Standard Azadiraction The abnormal adults formed
Fig 8d- m-Fluorobenzyl isobutylxanthate Oral consumption of the compound

**Detailed description of the invention**

The present invention particularly relates to develop Alkylxanthates as novel Insect Growth Regulators (IGR's) that are useful for integrated pest management. The alkylxanthates employed in the present invention are useful to maximize the lepidopteron pest infestation in agricultural fields with no or less environmental impact. These alkylxanthates as natural products are relatively uncommon in nature. We have isolated a Ethylene Bis (isobutylxanthate) (4) from a green alga Dictyosphaeria favulosa. It was tested against various aquatic stages of Aedes aegypti mosquito, which is a yellow fever vector. The compound 4 exhibited larvicidal and insect growth regulatory activity in dose dependent mannar. The analogues of Alkylxanthates has been proved as insect growth regulators (IGR) against two different lepidopteron pests (Spodoptera litura and Helicoverpa armigera), as similar to Azadiractin (standard compound) a natural product isolated from neem seed kernel. They have simple structures unlike Azadiractin and possessing same kind of mode of action, which can be synthesised easily in the laboratory.

The present invention provides three novel compounds of alkylxanthates namely:

(1) methylene Bis (tetrahydrofurfuryl xanthate)
(2) m-Fluorobenzyl n-butylxanthate and
(3) m-Fluorobenzyl isobutylxanthate having the structural formulae as shown in figures 1, 2 and 3 respectively of the accompanying drawings, for use in agricultural applications.

In an embodiment of the said compounds having the following properties on lepidopteron pest as:

(a) larvicidal,
(b) antifcedant, and
(c) insect growth regulator.

In still another embodiment the novel compounds possess toxicity towards brine shrimps and mosquitoes.

In still another embodiment of the invention, the novel compounds controls the growth of the agricultural pests *Spodoptera litura, and Helicover armigera.*

In yet another embodiment the novel compounds used as insect growth regulator is ranging from 50 μg to 100 μg per insect, (topical application on III instar larvae) and 5 to 25 μg oral feeding.

In yet another embodiment the larval mortality rate is in the range between 50 to 90 % when the above novel compounds are used.

In yet another embodiment of the invention, the amount of analogous of alkyl xanthates used for protecting the leafs are in the range between 15 to 50 micro grams per sq.cm of the leaf area.

In yet another embodiment, the novel compound methylene Bis (tetrahydrofurfuryl xanthate) 1 is a colourless liquid and the molecular formula is $C_{13}H_{20}O_4S_4$ and having the following properties:

| | |
|---|---|
| Physical state | Colourless liquid |
| Molecular formula | $C_{13}H_{20}O_4S_4$ |
| $^1$H NMR | ($CDCl_3$, 200 $MH_z$) δ1.70 (2H, m), 1.95 (2H,m), 3.71 (2H, m), 4.20 (1H, m), 4.55 (2H, t) and 4.78 (1H,s). |
| IR (Neat) | $\nu_{max}$ (KBr) 2920, 1470, 1235 and 1140 $cm^{-1}$ |
| EIMS (70 eV) | m/z 368 |
| UV (MeOH) | $\lambda_{max}$(MeOH) 217 (ε 11150), 242 (1650), 270 nm (19160) |

In yet another embodiment of the present invention, the novel compound m-fluorobenzyl n-butylxanthate 2 is a pale yellow liquid having molecular formula $C_{12}H_{15}FOS_2$ and having the following properties:

| | |
|---|---|
| Physical state | Pale yellow liquid |
| Molecular formula | $C_{12}H_{15}FOS_2$ |
| $^1H$ NMR | ($CDCl_3$, 200 $MH_z$) δ1.0 (3H, m), 1.42 (2H, m), 1.75 (2H, m), 4.60 (2H, t,*j* = 7.0 Hz), 4.35 (2H, s) and 6.95-7.20 (4H, m). |
| IR (Neat) | $\nu_{max}$ 2960, 2931,1615,1590,1460,1240 and 1045 $cm^{-1}$ |
| EIMS (70 eV) | m/z 258 |
| UV (MeOH) | $\lambda_{max}$ 210(ε 37105), 218 (35660) and 280 nm (27354) |

In yet another embodiment of the invention, the novel compound m-fluorobenzyl isobutylxanthate 3 is a pale yellow liquid and having molecular formula $C_{12}H_{15}FOS_2$ and having the following properties:

| | |
|---|---|
| Physical state | Pale yellow liquid |
| Molecular formula | $C_{12}H_{15}FOS_2$ |
| $^1H$ NMR | ($CDCl_3$, 200 $MH_z$) δ1.0 (6H, d, J = 7.0 Hz), 2.12 (1H, m), 4.34 (4H, brs), and 6.92-7.22 (4H, m). |
| IR (Neat) | $\nu_{max}$ 2964, 2937, 1620, 1585, 1472, 1230, 1165 and 1030 $cm^{-1}$ |
| EIMS (70 eV) | m/z 258 |
| UV (MeOH) | $\lambda_{max}$ 214(ε 37190), 220 (35674) and 274 nm (27378) |

One more embodiment of the invention provides a new use of analogues of alkylxanthates of formulae 1, 2, 3 and 5 of the drawings as insect growth regulators for the integrated pest management (IPM) and the analogues of alkylxanthates used are methylene bis (n-butyl xanthate), methylene bis (tetrafurfurylxanthate), m-fluorobenzyl n-butylxanthate and m-Fluorobenzyl isobutylxanthate, the structural formulae of the above compounds are shown in the figures 5, 1, 2 and 3 of the drawings respectively.

In still another embodiment of the invention, the alkyl xanthate controls the growth of the agricultural pests is *Spodoptera litura, and Helicover armigera.*

In still another embodiment the amount of alkylxanthates used as insect growth regulator is ranging from 50 μg to 100 μg per insect. (topical application on III instar larvae) and 5 to 25 μg oral feeding and the larval mortality rate is in the range between 50 to 90 %.

In yet another embodiment the analogues of alkylxanthates are used as an antifeedant for the integrated pest management, the analogues of alkylxanthates used are methylene bis (n-butyl xanthate), methylene bis (tetrafurfurylxanthate), m-Fluorobenzyl n-butylxanthate and m-Fluorobenzyl isobutylxanthate.

In yet another embodiment the amount of analogues of alkyl xanthates used for protecting the leafs are in the range between 15 to 50 micro grams per sq.cm of the leaf area.

In yet another embodiment of the invention, the alkyl xanthate acts against the ploy phagous pests, *spodotera litura* (Tabaco caterpillar) and controls the consumption of the leaf by the pests.

In further embodiment the present invention provides a process for the preparation of the analogues of alkyl xanthates useful as an antifeedant and as insect growth regulators, said process comprising:

(a) refluxing, KOH with an alcohol followed by adding carbondisulphide to get a yellow crystalline compound namely potassium -O-alkylxanthate,

(b) reacting the potassium-O-alkylxanthate in the presence of ketone with a bromide derivative selected from methylene dibromide or m-fluorobenzyl bromide, filtering and removing the ketone under reduced pressure to obtain corresponding alkylxanthate compounds 1, 2, 3 and 5 with the yield ranging from 70 to 80 %.

In still another embodiment refluxing is carried out at a 0°C.

In one more embodiment of the invention provides a novel composition used as an insect growth regulator and an antifeedant for the integrated pest management, said composition comprising an effective amount of alkylxanthate and analogues of alkylxanthates with an additive.

The analogues of alkyl xanthate are selected from methylene bis (n-butyl xanthate), methylene bis (tetrahydrofurfuryl xanthate), m-Fluorobenzyl n-butylxanthate and m-Fluorobenzyl isobutylxanthate or mixture thereof.

In still another embodiment the additive is a solvent, i.e. $C_9$, cycloxonone, acetone, ethyl methyl ketone etc., with emulsifier piperonyl butoxide (5%).

In yet another embodiment, the ratio of the additive to the active ingredients is in the ratio ranging from 80 to

95%.

In yet another embodiment, the amount required for controlling the antifeedant activity is 30 to 60 µg/sq.cm.

In yet another embodiment, the amount required for use as insect growth regulators activity is 50 to 100 µg/insect (Topical) or 5to 25 µg oral feeding.

The following examples are given merely to understand the invention clearly and these examples should not be construed to limit the scope of invention in any manner.

**Examples**

Preparation of bis (alkylxanthates): Into a 100-ml round-bottomed flask, fitted with a reflux condenser, placed 1.05 gm (0.0187 mol) of KOH pellets was added excess amount (0.072 mol) of alcohol and the reaction mixture was refluxed for 1 hr. The reaction mixture was cooled and decanted the liquid from the residual solid into another dry 100-ml R.B flask.

To this cold reaction mixture 1.37 gm (0.0187 mol, 1.08 ml) of carbon disulfide was added slowly with constant stirring to yield yellow crystalline compound potassium-O-alkylxanthate (yield 80-90%).

To the above potassium-O-alkylxanthate (0.01mol) in a 50 ml of acetone was added methylenedibromide 0.869 gm (0.348 ml, 0.005 mol) or ethylenedibromide 0.939 gm (0.52 ml, 0.005 mol) and refluxed for one hour. The reaction sets in within 15 minutes and the yellow reaction mixture becomes white owing to the separation of KBr. Then the reaction mixture was filtered, acetone was removed under vacuum. After usual workup afforded bis (alkylxanthate)

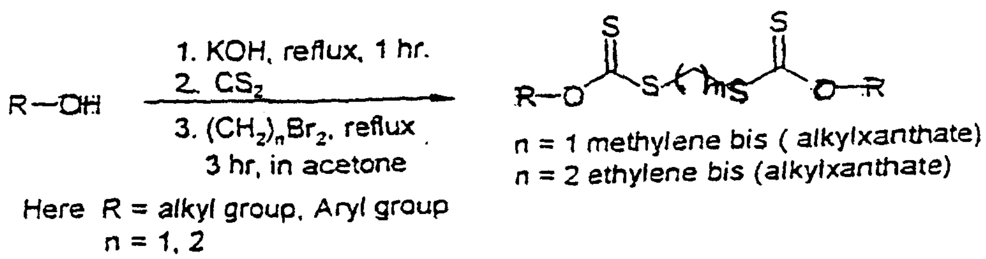


(yield 75 to 90%) showed in **Table 1**.

Table: 1

| Compound No. | Compound Name | R | n |
|---|---|---|---|
| **4** | **Ethylene Bis (isobutylxanthate)** | **Isobutyl** | **2** |
| **5** | **Methylene Bis (n-butytxanthate)** | **n-butyl** | **1** |
| **23** | **Ethylene Bis ($2^0$-butylxanthate)** | **$2^0$-butyl** | **2** |
| **24** | **Methylene Bis ($2^0$-butylxanthate)** | **$2^0$-butyl** | **1** |
| **5'** | **Ethylene Bis (benzylxanthate)** | **Benzyl** | **2** |
| **3'** | **Ethylene Bis (n-butylxanthate)** | **n-butyl** | **2** |
| **1** | **Methylene Bis (tetrahydrofurfurylxanthate)** | **Tetrahydro furfuryl** | **1** |

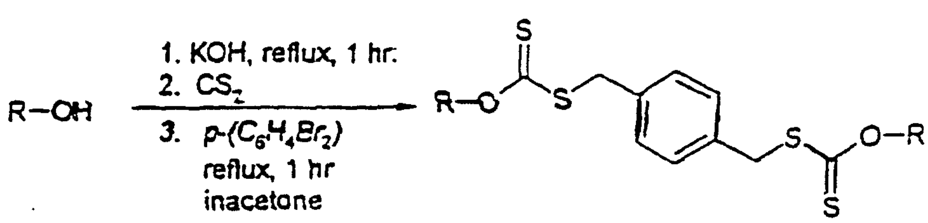


Here R = alkyl group

Preparation of p-xylene bis (alkylxanthates): A mixture of potassium-O-alkylxanthate (0.01 mol) and p-xylene dibromide (0.005 mol) in a 50 ml of acetone refluxed for one hour. The reaction sets in within 15 minutes and the yellow reaction mixture becomes white owing to the separation ofKBr. Then the reaction mixture was filtered, acetone was removed under vacuum. After usual workup yielded p-xylene bis (alkylxanthate) (yield 75-85 %) showed in **Table 2.**

Table: 2

| Compound No. | Compound Name | R |
|---|---|---|
| **12 (Fig .6 )** | **p-Xylene Bis (n-butylcanthate)** | **n-butyl** |
| **26** | **P-Xylene Bis (2$^0$butylxanthate)** | **2°-butyl** |

Preparation of m-fluorobenzyl alkylxanthates: A mixture of potassium-O-alkylxanthate (0.01 mol) and m-fluorobenzyl bromide (0.01 mol) in a 50 ml of acetone refluxed for one hour. The reaction sets in within 15 minutes and the yellow reaction mixture becomes white owing to the separation of KBr. Then the reaction mixture was filtered, acetone was removed under vacuum. After usual workup yielded m-fluorobenzyl alkylxanthate (yield 70-80 %) showed in Table 3.

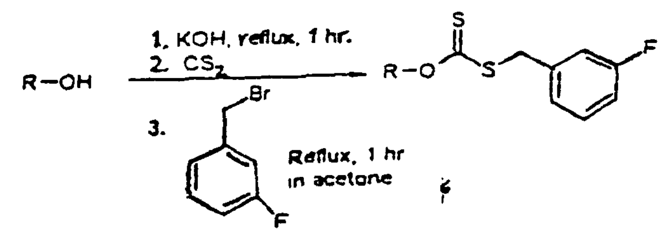


Here R = alkyl group

Table: 3

| Compound No. | Compound Name | R |
|---|---|---|
| **2** | **m-Fluorobenzyl (n-butylxanthate)** | **n-butyl** |
| **3** | **m-Fluorobenzyl (isobutylxanthate)** | **Isobutyl** |

Preparation of organophosphorus compounds: A mixture of potassium-O-alkylxanthate (0.01 mol) and O,O-diethyl thio phosphonyl chloride (0.01 mol) in a 50 ml of acetone refluxed for one hour. The reaction sets in within 15 minutes and the yellow reaction mixture becomes white owing to the separation of KCl. Then the reaction mixture was filtered, acetone was removed under vacuum. After usual workup yielded O,O-diethyl thiophosphate alkylxanthate (yield 70-80 %) showed in Table 4.

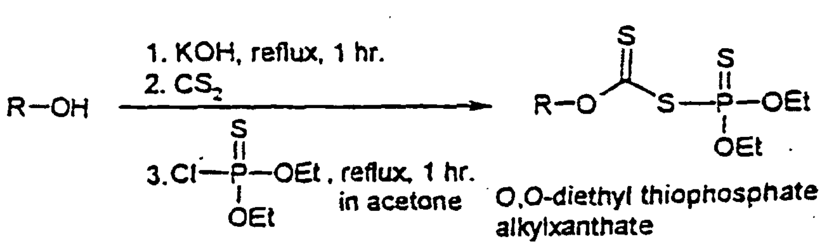


Here R = alkyl group, aryl group

Table: 4

| Compound No. | Compound Name | R |
|---|---|---|
| **29** | **O,O-diethyl thiophospate isobutylxanthate** | **Isobutyl** |

Table: 4 (continued)

| Compound No. | Compound Name | R |
|---|---|---|
| **30** | **O,O-diethyl thiophospate $2^0$-butylxanthate** | **$2^0$-butyl** |
| **10** | **O,O-diethyl thiophospate n-butylxanthate** | **n-butyl** |
| **31** | **O,O-diethyl thiophospate phenethylxanthate** | **Phenethyl** |

Biological activities

Methodology for Larvicidal activity:

To determine the efficacy of different analogues of Alkylxanthates against mosquito larvae, standard procedures as recommended by World Health Organization (1981) were followed with minor modifications. The desired concentration of the test solution was obtained by adding 1 ml of an appropriate stock solution to 249 ml of tap water in 500 ml beaker. Test solutions of different concentrations were prepared by diluting the stock solution in dechlorinated and filtered tap water followed by thorough mixing. Tween -80 [Poly (oxyethylene)$_n$ - sorbitan monooleate] was used as an emulsifier at the concentration of 0.002% (v/v) to the final test solution whenever required. Control solutions were prepared in tapwater using acetone or ethanol and Tween -80 (0.002%). To each of the -test concentrations, 25 late third or early fourth instar larvae which were acclimatized earlier were added. At least three replicates of each of the tests were made. Based on the results, five to ten suitable concentrations, which produced 10 to 100% mortality, were selected for detailed study and for calculating $LC_{50}$ values. Mortality was observed 24 hours after exposing the larvaeto test solutions and the data was corrected for control mortality if any, by using Abbot's formula (Abbot 1925). All tests were carried out at a room temperature maintained at 28 ± 1°C.

Methodology For Developmental Inhibition On Mosquitoes:

The developmental inhibition potential of different analogues of Alkylxanthates a evaluated based on the method of Mulla et al., 1974. Experiments were carried out with second, third, fourth instar larvae and pupae. Initially, to each of the 500 ml beakers containing wide range of test concentrations, 25 larvae were added. Based on the results, five to ten suitable concentrations, which produced 10 to 100% growth inhibition, were selected for detailed study. The data of atleast three to four replicates each of three separate experiments was subjected to statistical analysis. Observations on abnormalities in larvae, pupae and adult emergence were made until 100% emergence in controls. All the experiments were performed at room temperature.

BRINE SHRIMP BIOASSAY:

Brine shrimp (*Artemiasalina*) eggs were hatched in a shallow rectangular dish filled with artificial seawater, which was prepared with commercial salt mixture and double distilled water. A plastic divider with several 2-mm holes was clamped in the dish to make two unequal compartments. The eggs were sprinkled into the larger compartment that was darkened, while the smaller compartment was illuminated. After 48 hours the phototropic nauplii were collected by pipette from the lighted side, being separated by the divider from their shells.

Twenty shrimps were transferred to each sample vial and artificial seawater was added to make 5 ml. The nauplii can becounted macroscopically in the stem of the pipette against a lighted back ground. A drop of dry yeast suspension (3µg in5ml artificial seawater) was added as food to each vial (Meyer, et al., 1982). Survivors were counted with the aid of a 3 x magnifying glass, after 24 hours, and the percent mortality at each concentration and control was determined. The 24 hour counts were more useful. During control deaths, the data was corrected using Abbot's formula.

STATISTICAL ANALYSIS

The average mortality and growth inhibition data of experiments was subjected to IBM AT 386 computer analysis at our laboratory for calculating $LC_{50}$, $LD_{50}$, $EC_{50}$, $ED_{50}$,etc., and other statistical data. The software used was developed by Reddy et al., 1992.

Methodology for Antifeedant activity: Tobacco caterpillar, *Spodoptera litura F.,* larvae will be reared on *Ricinus cummunis L.* leaves at 27°C and 65-70% R.H. Known area ($cm^2$) of leaf disks were painted with exact amount of pure compound (dissolved in acetone) and were dried at room temperature till the carrier solvent evaporated. For each concentration 5 to 6 replicates were prepared and experiments were conducted individually. Control disks were dipped

in carrier solvent (acetone) alone. Third instar larvae weighing 200-250 mg are selected from stock culture, starved for 4 hr, and will be used individually for the assay of antifeedant activity. After 12 and 24 hours, leaf area meter was used to measure the consumed area of treated and untreated leaves. Based on the data the percent protection of individual compound was calculated. All these properties were compared with well-known antifeedant isolated from neem kemal 'Azadiractin'. Control and test larvae arc transferred on fresh leaves and'monitored till adult emergence to study the growth and behavioral aspects of the insect.

Methodology for Growth regulatory activity: In continuation to Anteefedant experiments, the larvae (leaf consumed along with compound-calculated) were individually monitored till adult emergence. Based on the formation of normal, abnormal pupae, normal adult emergence and abnormal adult emergence were recorded and calculated the growth regulatory activity of the compound in comparison with standard. Relative inhibition 50 values of alkylxanthates on *spodoptera litura* and *Helicoverpa armigera* were statistically analysed.

**Results**

1. Larvicidal activity of xanthates and its analogues against IV instar larvae of *Aedes aegypti*

| S. No. | Compound | $LC_{50} \pm$ S.E. (mg/l) |
|---|---|---|
| 1. | Ethylene bis (isobutylxanthate) | 8.06 ± 0.22 |
| 2. | Methylene bis (n-butylxanthate) | 5.33 ± 0.97 |
| 3. | Methylene bix (tetrahydrofurfurylxanthate) | 3.77 ± 0.59 |
| 4. | p-Xylene bis (n-butylxanthate) | 7.35 ± 0.51 |
| 5. | m-Fluorobenzyl n-butylxanthate | 12.53 ± 1.24 |
| 6. | m-Fluorobenzyl isobutylxanthate | 11.78 ± 2.35 |

II. Developmental Inhibition activity of xanthates and its analogues against third instar larvae *of Aedes aegypti*

The sub lethal concentrations of all the analogues [$LC_{10}$ to $LC_{20}$ concentrations] have exhibited developmental inhibition on mosquito, *Aedes aegypti.* Abnormal pupalformation and pupal case attachment to emerged adults (leads to mortality) were quite common in most of the xanthates.

III. Toxic activity of xanthates and its analogues against Brine shrimp, *Artemia salina*

| S.No. | Compound | $LC_{50} \pm$ S.E.(mg/ l) |
|---|---|---|
| 1. | Ethylene bis (isobutylxanthate) | 25.78 ± 2.90 |
| 2. | Methylene bis(n-butylxanthate) | 34.65 ± 3.91 |
| 3. | Methylenebis(tetrahydrofurfurylxanthate) | 5.63 ±0.62 |
| 4. | Xylene bis(n-butylxanthate) | 45.23 ± 5.63 |
| 5. | m-fluorobenzyl n-butylxanthate | 19.00 ± 2.83 |
| 6. | m-Fluorobenzyl isobutylxanthate | 22.54 ±3.26 |
| 7. | Standard 'Chamtothecin' | 2.53 ± 0.43 |

IV. Antifeedant activity of xanthate and its analogues against late third instar of *Spodoptera litura*

| Compound code | Conc. in μg/sq.cm. | Percent leaf consumption | | | Concentration Concentration leaf required for 50% leaf protection |
|---|---|---|---|---|---|
| | | 12 hr | 24 hr | A.I | $EC_{50}$ in $\mu g/cm^2 \pm$S.E |
| Standard Azadiractin | **20** | **4.2** | **12.7** | **76.42** | 6.34±.77 |
| | **15** | **16.4** | **27.6** | **54.98** | |
| | **10** | **18.6** | **33.3** | **48.09** | |
| | **5.0** | **33.7** | **57.2** | **24.84** | |
| Ethylene Bis (isobutylxanthate) | **65** | **9.3** | **25.2** | **58.07** | 42.46±5.76 |
| | **50** | **22.4** | **45.9** | **34.85** | |
| | **35** | **36.1** | **62.7** | **20.48** | |
| | **20** | **60.2** | **80.7** | **8.14** | |
| Methylene Bis (n-butylxanthate) | **30** | **-0-** | **7.5** | **85.37** | 17.11±1.36 |
| | **25** | **3.5** | **20.7** | **64.22** | |
| | **20** | **22.5** | **45.6** | **35.14** | |
| | **15** | **35.2** | **60.4** | **22.27** | |
| | **10** | **48.5** | **85.1** | **5.50** | |
| Methylene Bis (tetrahydrofurfuryl alcohol) | **50** | **22.1** | **45.5** | **35.23** | 37.21±9.67 |
| | **35** | **25.9** | **47.3** | **33.52** | |
| | **25** | **31.5** | **60.2** | **22.42** | |
| | **12.5** | **48.6** | **80.6** | **8.20** | |
| p-Xylene Bis (n-butylxanthate) | **25** | **20.5** | **24.6** | **58.86** | 20.45±1.76 |
| | **20** | **35.2** | **57.6** | **24.51** | |
| | **15** | **62.4** | **84.3** | **5.97** | |
| | **10** | **57.3** | **90.2** | **2.59** | |
| m-Fluorobenzyl isobutylxanthate | **25** | **12.5** | **32.8** | **48.67** | 19.65±2.99 |
| | **20** | **24.7** | **53.6** | **27.86** | |
| | **15** | **33.24** | **68.7** | **16.07** | |
| | **10** | **50.4** | **75.2** | **11.63** | |

Data represented as the mean of five replicates in three different sets of experiments.

$$\text{A.I=Anti-feedent index} = \frac{\text{(Control consumption) - (Treated consumption)}}{\text{(Control consumption) + (Treated consumption)}} \text{ X } 100$$

**Normal and control worms consumed 45-50% leaf in 12 hours and 87-95% leaf consumption in 24 hours.**

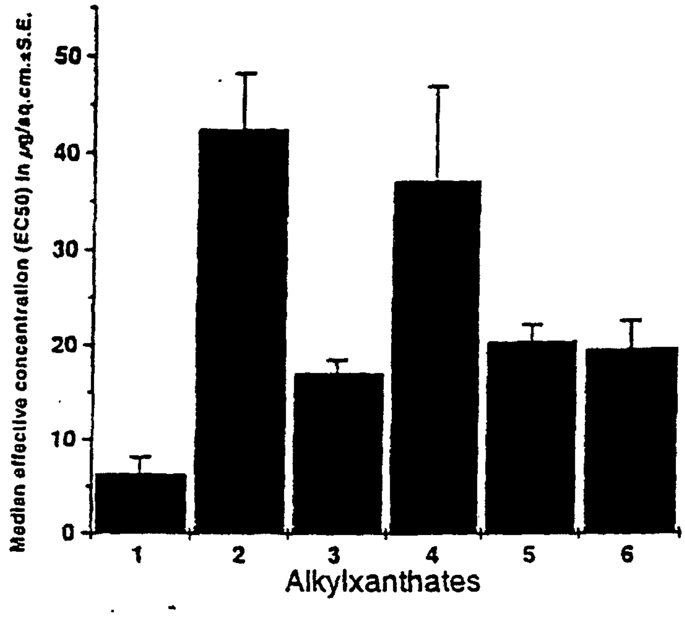


1. Standard Compound
2. Ethylene bis (isobutylxanthate)
3. Methylene bis (n-butylxanthate)
4. Methylene bis (tetrahydrofurfurylxanthate)
5. p-Xylene bis (n-butylxanthate)
6. m-Fluorobenzyl isobutylxanthate

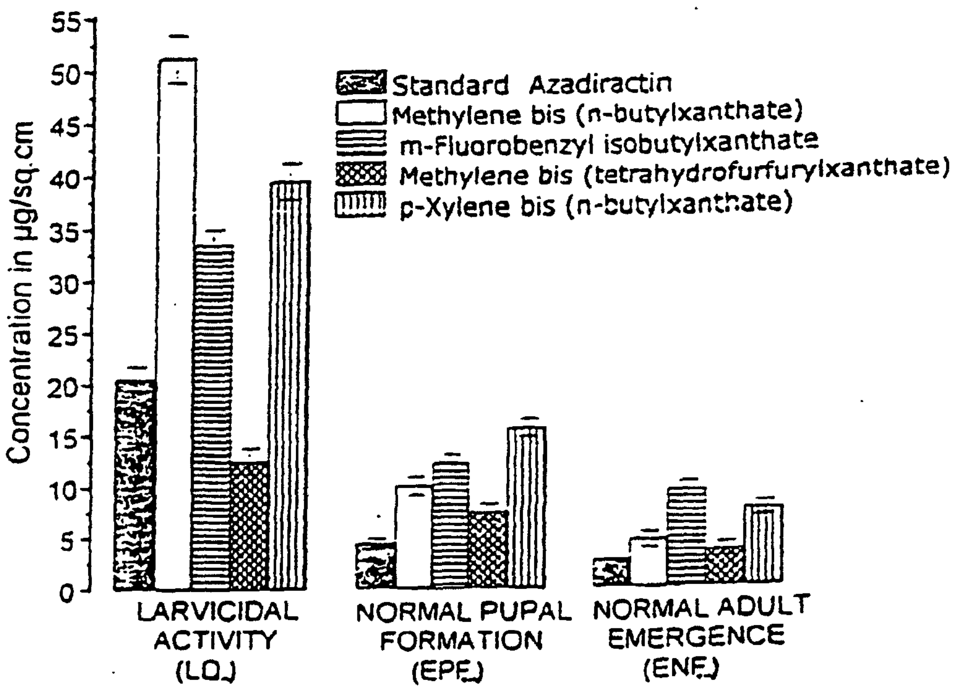

It is concluded that the above alkylxanthates have exhibited similar kind of mode of action as Azadiractin. Though alkylxanthates have less active than Azadiractin but they can be very easily synthasized and possessing quick bio-degradability. The above results were obtained through oral feeding. Similar activities were also observed when these larvae were treated topically with known amount of compounds. These experiments are also conformed that the mode of action is similar to Azadiractin.

Bio-evaluations on *Helicoverpa armigera (an* Agricultural pest) Topical application of analogues).

Our earlier studies exhibited the analogues of alkylxanthates as an antifeedents and insect growth regulator properties against agricultural pest *Spodoptera litura.*

Further experiments were performed on *Helicoverpa armigera (Hubner),* a lepidopteron pest was used as an experimental modal to reconfirm the IGR activities of alkylxanthates. Once again the present results proved that the importance of these compounds as potential candidates as Insect Growth Regulators (IGR) against lepidopteron pests. The activity of Azadiractin (standard compound) and alkylxanthates has a similar mode of action i.e., less larvicidal activity, minimized in the formation of normal pupae and emergence of normal adults.

The genera Helicoverpa (Heliothis) is one of the most important Lepidopteron pest of both subsistence and cash crops in the new and old worlds. *Helicoverpa armigera (Hubner),* commonly referred to as the American bollworm or gram pod borer, is the most widely distributed of the group occurring in Africa, Asia, Australia, Oceania and Europe. The polyphagous larvae attack cotton, maize, sorghum, sunflower, tomato, okra and a range of legumes.

In India, annual loses to pigeonpea and chickpea alone may exceed US$ 300 million (Reed and power, 1982), and a more recent estimate by Mehrotra (Indian Agricultural Research Institute, unpublished) suggests that total loses to pluses and cotton are likely to be more than US$ 530 million per annum.

V

| Sample Code | Conc. (µg/ Insect) | No. insects used for assay | Larval morta-lity (%) | PUPAL FORMATION | | | Adult emergence | | | |
|---|---|---|---|---|---|---|---|---|---|---|
| | | | | Normal pupa | Abnormal Pupa | Pupal Mortality | Normal adults | Abnormal adults | Inter-mediates | Non emer-gence |
| Methylene Bis (n-butylxanthate). | 100 | 20x5 | 61 | -0- | 22 | 17 | -0- | -0- | -2- | 20 |
| | 75 | 20x5 | 49 | -0- | 27 | 24 | -0- | -1- | -2- | 24 |
| | 50 | 20x5 | 28 | 12 | 32 | 28 | -8- | -9- | -6- | 21 |
| | 35 | 20x5 | 12 | 27 | 28 | 33 | 17 | 11 | 18 | -9- |
| | 20 | 20x5 | -7- | 52 | 36 | -5- | 27 | 41 | 17 | -3- |
| O,O-diethyl thiophospate phenethyl xanthate | 100 | 25x4 | 92 | -0- | -6- | -3- | -0- | -1- | -0- | -5- |
| | 75 | 25x4 | 85 | -3- | -5- | -7- | -4- | -1- | -0- | -3- |
| | 50 | 25x4 | 71 | -5- | 11 | -9- | -5- | -7- | -2- | -2- |
| | 35 | 25x4 | 62 | 12 | 15 | 11 | -9- | 16 | -4- | -8- |
| | 25 | 25x4 | 47 | 23 | 13 | 17 | 15 | -7- | -5- | -9- |
| p-Xylene Bis (n-butylxanthate) | 100 | 20x5 | 85 | -0- | -6- | 15 | -0- | -1- | -1- | -4- |
| | 75 | 20x5 | 71 | -5- | -7- | 17 | -0- | -2- | -2- | -8- |
| | 50 | 20x5 | 56 | 10 | 13 | 21 | -3- | 13 | -3- | -4- |
| | 35 | 20x5 | 42 | 12 | 18 | 28 | -9- | 12 | -3- | -6- |
| | 20 | 20x5 | 18 | 39 | 27 | 16 | 33 | 19 | -7- | -5- |
| Control | 100 | 25x4 | 3 | 97 | -0- | -0- | 94 | -0- | -0- | -3- |
| Azadiractin | 50 | 25x4 | 91 | -0- | -5- | -4- | -0- | -2- | -2- | -1- |
| | 35 | 25x4 | 68 | -8- | 12 | 12 | -2- | -6- | -4- | -3- |
| | 20 | 25x4 | 47 | 13 | 27 | 13 | 10 | 17 | -5- | -8- |
| | 10 | 25x4 | 26 | 33 | 23 | 18 | 18 | 21 | 10 | -7- |
| | 5 | 25x4 | 11 | 47 | 31 | 11 | 38 | 18 | 20 | -2- |
| | 2.5 | 25x4 | -2- | 62 | 28 | -8- | 51 | 26 | 13 | -0- |

The analogues of alkylxanthates has been proved as insect growth regulators (IGR) against two different lepidopteron pests as similar to Azadiractin (standard compound) a natural product isolated from neem seed kernel. They have simple structures unlike Azadiractin and possessing same kind of mode of action, which can be synthesized easily in the laboratory.

VI. Bio-evaluations on Agricultural pest, *Spodoptera litura* (Topical application of xanthate analogues)

| Sample Code | Conc.(μg/insect) | No. insectsused for assay | Larval mortality(%) | PUPAL FORMATION | | | ADULT EMERGENCE | | | |
|---|---|---|---|---|---|---|---|---|---|---|
| | | | | Nomal pupa | Abnormal pupa | Pupal Mortality | Normal adults | Abnormal adults | Intermediates | Non-emergence |
| Methylene bis(n-butylxanthate) | 100 | 20x5 | 86 | -0- | 14 | 14 | -0- | -0- | -0- | 14 |
| | 75 | 20x5 | 72 | -0- | 28 | 25 | -0- | -1- | -2- | 25 |
| | 50 | 20x5 | 57 | 5 | 38 | 24 | 5 | 6 | 8 | 9 |
| | 35 | 20x5 | 21 | 25 | 54 | 26 | 7 | 23 | 23 | 25 |
| | 20 | 20x5 | 0 | 47 | 53 | 17 | 24 | 27 | 32 | -2- |
| | 10 | 20x5 | 0 | 52 | 48 | -8- | 36 | 22 | 34 | -1- |
| | 5 | 20x5 | 0 | 59 | 41 | -5- | 41 | 26 | 28 | -0- |
| M-Fluoro benzyl isobutyl xanthate | 100 | 20x5 | 96 | -0- | -4- | -4- | -0- | -0- | -0- | -0- |
| | 75 | 20x5 | 83 | -9- | -8- | 13 | -0- | -3- | -1- | -2- |
| | 50 | 20x5 | 70 | 13 | 15 | -9- | -3- | 11 | -5- | -4- |
| | 35 | 20 x 5 | 56 | 25 | 19 | -7- | 17 | 14 | -6- | -0- |
| | 20 | 20 x 5 | 27 | 43 | 30 | -4- | 39 | 22 | -8- | -0- |
| | 10 | 20 x 5 | 10 | 58 | 32 | -8- | 49 | 29 | -4- | -1- |
| | 5 | 20 x 5 | 3 | 73 | 24 | -3- | 50 | 32 | -2- | -0- |
| Methylene bis (tetrahydrofurfury lxanthate | 100 | 25 X 4 | 98 | -0- | -2- | -1- | -0- | -1- | -0- | -0- |
| | 75 | 25 X 4 | 91 | -2- | -7- | -5- | -2- | -2- | -0- | -2- |
| | 50 | 25 X 4 | 80 | -7- | 13 | -4- | -4- | -9- | -3- | -1- |
| | 35 | 25 X 4 | 71 | 19 | 10 | -7- | -9- | 11 | -2- | -3- |
| | 25 | 25 X 4 | 59 | 25 | 16 | -7- | 15 | 17 | -2- | -2- |
| | 10 | 25 X 4 | 46 | 44 | 10 | -6- | 28 | 19 | -1- | -2- |
| | 5 | 25 X 4 | 36 | 58 | 6 | -5- | 37 | 22 | -0- | -3- |
| p-Xylene bis (n-butylxanthate) | 100 | 20 X 5 | 95 | -0- | -5- | -4- | -0- | -0- | -1- | -2- |
| | 75 | 20 X 5 | 86 | -3- | -11- | 10 | -0- | -2- | -2- | -2- |
| | 50 | 20 X 5 | 71 | -8- | 21 | 14 | -2- | 13 | -3- | -1- |
| | 35 | 20 X 5 | 59 | 12 | 29 | 13 | -9- | 17 | -2- | -4- |
| | 20 | 20 X 5 | 20 | 59 | 21 | -5- | 25 | 36 | -14- | -5- |
| | 10 | 20 X 5 | 5 | 72 | 23 | -3- | 44 | 38 | 10 | -1- |
| | 5 | 20 X 5 | -0- | 85 | 15 | -2- | 59 | 29 | 10 | -0- |
| Control | 100 | 25X4 | 5 | 95 | -0- | -0- | 94 | -0- | -0- | -1- |

| | | | | | | | | | | |
|---|---|---|---|---|---|---|---|---|---|---|
| Standard Azadiractin | 75 | 25X4 | 96 | -0- | -4- | -4- | -0- | -0- | -0- | -0- |
| | 50 | 25X4 | 88 | -7- | -5- | -8- | -0- | -2- | -2- | -1- |
| | 35 | 25X4 | 72 | -10- | 18 | 16 | -2- | -6- | -4- | -2- |
| | 20 | 25X4 | 52 | 19 | 29 | 10 | 10 | 17 | 11 | -3- |
| | 10 | 25X4 | 30 | 38 | 32 | -7- | 28 | 21 | 14 | -1- |
| | 5 | 25X4 | -9- | 45 | 34 | 11 | 38 | 19 | 20 | -2- |
| | 2.5 | 25X4 | -0- | 62 | 38 | -6- | 47 | 26 | 21 | -6- |

## Claims

**1.** Akylxanthate compounds, namely:

(1) methylene Bis (tetrahydrofurfuryl xanthate),
(2) m-Fluorobenzyl n-butylxanthate, and
(3) m-Fluorobenzyl isobutylxanthate

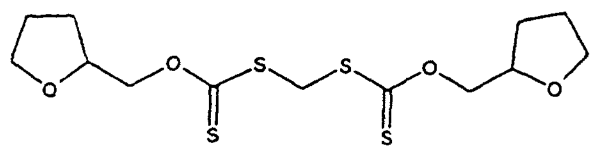

1

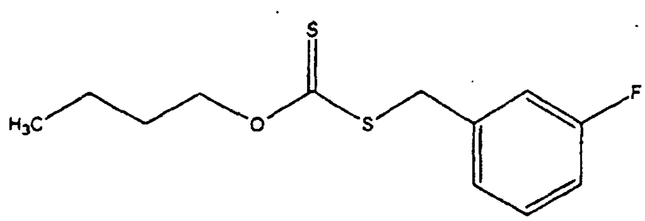

2

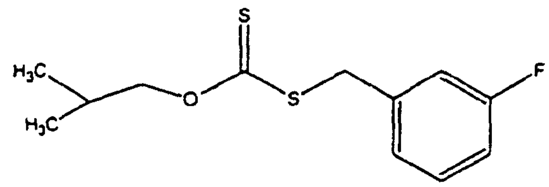

3

2. Use of analogues of alkylxanthates as insect growth regulators for the integrated pest management (IPM), the said analogues being selected from a group consisting of methylene bis (tetrahydrofurfurylxanthate), m-fluorobenzyl n-butylxanthate, m-fluorobenzyl isobutylxanthate or methylene bis (n-butylxanthate), represented by formulae 1,2,3 and 5 respectively.

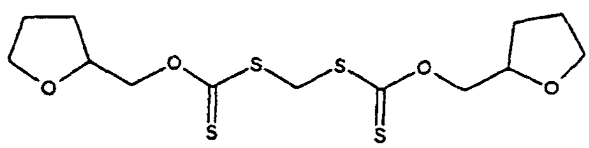

1

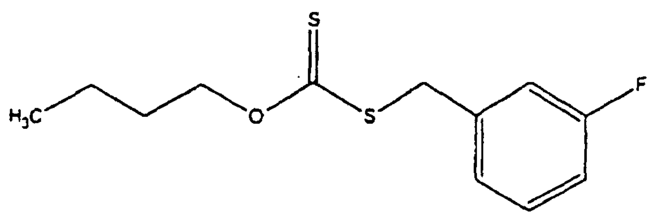

2

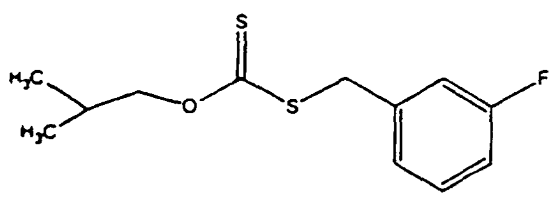

3

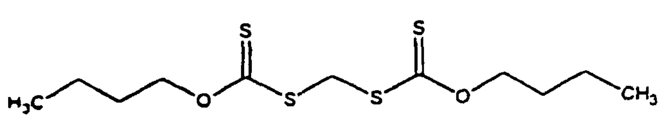

5

3. Use as claimed in claim 2 wherein, the alkylxanthate controls the growth of the agricultural pests selected from

the group consisting of *Spodoprera litura* and *Helicover armigera*.

**4.** Use as claimed in claim 2 wherein the amount of alkylxanthates used as insect growth regulator is ranging from 50 μg to 100 μg per insect for topical application on III instar larvae and 5 to 25 μg for oral feeding.

**5.** Use as claimed in claim 2 wherein the larval mortality rate is in the range between 50 to 90 %.

**6.** Use of analogues of alkylxanthates as an antifeedant for the integrated pest management, the said analogues being selected from methylene bis (tetrahydrofurfurylxanthate), m-Fluorobenzyl n-butylxanthate, m-Fluorobenzyl isobutylxanthate or methylene bis (n-butyl xanthate) and represented by chemical formulae 1,2,3 and 5 as shown in claim 2.

**7.** Use as claimed in claim 6 wherein the amount of analogues of alkyl xanthates used for protecting the leaf are in the range between 15 to 50 micro grams per sq.cm of the leaf area.

**8.** Use as claimed in claim 6 wherein the alkyl xanthate acts against the ployphagous pests, *spodotera lititra* (Tabaco caterpillar) and controls the consumption of the leaf by the pests.

**9.** A process for the preparation of analogues of alkyl xanthates as defined in claim 2 the said process comprising the steps of:

a. refluxing KOH with an alcohol followed by adding carbondisulphide to get a yellow crystalline compound, namely potassium -O-alkylxanthate,
b. reacting the potassium-O-alkylxanthate in the presence of ketonic solvent with a bromide derivative selected from methylene dibromide or m-fluorobenzyl bromide, filtering and removing the ketone under reduced pressure to obtain the desired alkylxanthate compounds.

**10.** A process as claimed in claim 9 wherein the reaction is carried out at 0°C.

**11.** A Composition comprising an effective amount of an alkylxanthate analogue selected from methylene bis (tetrahydrofurfuryl xanthate), m-Fluorobenzyl n-butylxanthate, m-Fluorobenzyl isobutylxanthate or methylene bis (n-butyl xanthate) of formulae 1, 2, 3 and 5 as shown in claim 2, or mixtures thereof, with an additive.

**12.** A composition as claimed in claim 11 wherein the additive is a solvent, i.e. C-IX, cycloxonone, acetone, ethyl methyl ketone etc., with emulsifier piperonyl butoxide (5%).

**13.** A composition as claimed in claim 11 wherein the ratio of the additive to the active ingredients is ranging from 80 to 95%.

**14.** A composition as claimed in claim 11 wherein the amount required for controlling the antifeedant activity is in the range of 30 to 60 μg/sq.cm.

**15.** A composition as claimed in claim 11 wherein the amount required for use as insect growth regulator activity is in the range of 50 to 100 μg/insect for topical applications or 5 to 25 μg for oral feeding.

**Patentansprüche**

**1.** Alkylxanthat-Verbindungen, nämlich:

(1) Methylen-bis-(tetrahydrofurfurylxanthat),
(2) m-Fluorbenzyl-n-butylxanthat, und
(3) m-Fluorbenzyl-isobutylxanthat

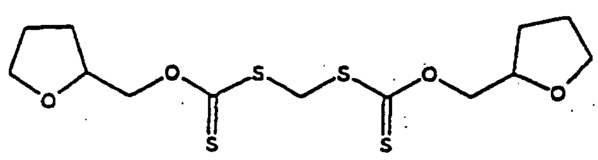

1

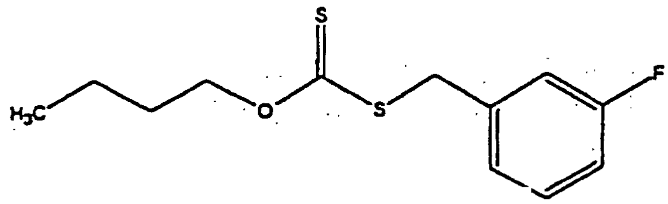

2

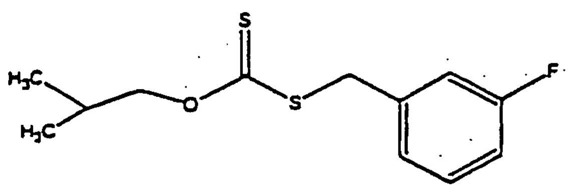

3

2. Verwendung von Analogen von Alkylxanthaten als Insektenwachstumsregulatoren für integriertes Schädlingsbekämpfungsmanagement (IPM), wobei die besagten Analoge aus einer Gruppe ausgewählt sind, bestehend aus Methylen-bis-(tetrahydrofurfurylxanthat), m-Fluorbenzyl-n-butylxanthat, m-Fluorbenzyl-isobutylxanthat oder Methylen-bis-(nbutylxanthat), dargestellt durch Formeln 1, 2, 3 bzw. 5.

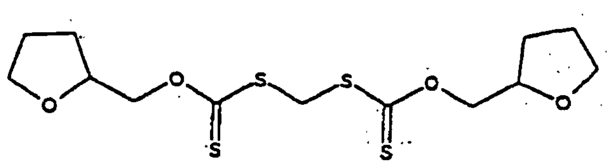

1

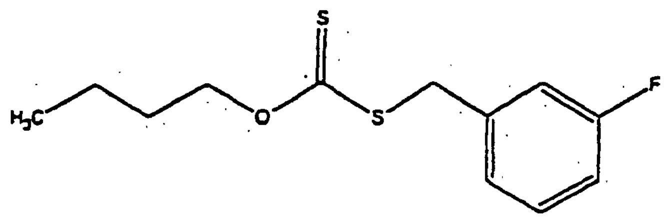

2

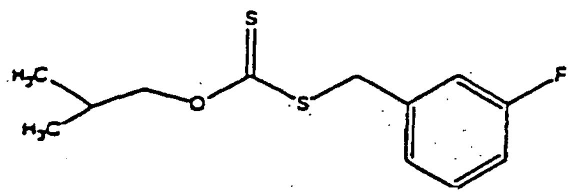

3

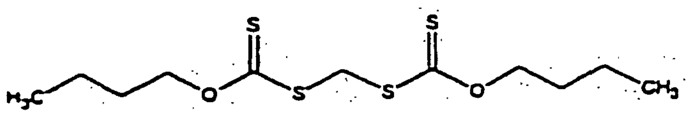

5

**3.** Verwendung nach Anspruch 2, wobei das Alkylxanthat das Wachstum der landwirtschaftlichen Schädlinge kontrolliert, ausgewählt aus der Gruppe, bestehend aus *Spodoptera litura* und *Helicover armigera.*

**4.** Verwendung nach Anspruch 2, wobei die Menge an als Insektenwachstumsregulator verwendeten Alkylxanthaten im Bereich von 50 µg bis 100 µg pro Insekt für die topische Aufbringung auf Larven im Larvenstadium III und 5 bis 25 µg zur oralen Verfütterung beträgt.

**5.** Verwendung nach Anspruch 2, wobei die Larven-Sterblichkeitsrate im Bereich zwischen 50 bis 90% liegt.

**6.** Verwendung von Analogen von Alkylxanthaten als fraßverhinderndes Mittel für das integrierte Schädlingsbekämpfungsmanagement, wobei die besagten Analoge ausgewählt sind aus Methylen-bis-(tetrahydrofurfurylxanthat), m-Fluorbenzyl-n-butylxanthat, m-Fluorbenzyl-isobutylxanthat oder Methylen-bis-(n-butylxanthat) und durch die chemischen Formeln 1, 2, 3 und 5, die in Anspruch 2 gezeigt sind, dargestellt sind.

**7.** Verwendung nach Anspruch 6, wobei die Menge an Analogen von Alkylxanthaten, die zum Schützen des Blattes verwendet werden, im Bereich zwischen 15 bis 50 Mikrogram pro $cm^2$ der Blattfläche beträgt.

**8.** Verwendung nach Anspruch 6, wobei das Alkylxanthat gegen polyphage Schädlinge, *Spodoptera litura* (Tabak-Raupe) wirkt, und den Verzehr des Blattes durch die Schädlinge kontrolliert.

**9.** Verfahren zur Herstellung von Analogen von Alkylxanthaten, wie in Anspruch 2 definiert, wobei der besagte Prozeß die Schritte umfaßt:

a. Refluxieren von KOH mit einem Alkohol, gefolgt von der Zugabe von Kohlenstoffdisulphid, um eine gelbe kristalline Verbindung, nämlich Kalium-O-Alkylxanthat, zu erhalten,

b. Umsetzen des Kalium-O-Alkylxanthats in der Anwesenheit eines ketonischen Lösungsmittels mit einem Bromidderivat, ausgewählt aus Methylenbromid oder m-Fluorbenzylbromid, Filtern und Entfernen des Ketons unter verringertem Druck, um die erwünschten Alkylxanthat-Verbindungen zu erhalten.

**10.** Verfahren nach Anspruch 9, wobei die Reaktion bei 0°C durchgeführt wird.

**11.** Zusammensetzung, umfassend eine wirksame Menge eines Alkylxanthat-Analogs, ausgewählt aus Methylen-bis-(tetrahydrofurfurylxanthat), m-Fluorbenzyl-n-butylxanthat, m-Fluorbenzyl-isobutylxanthat oder Methylen-bis-(n-butylxanthat) der Formeln 1, 2, 3 und 5, wie in Anspruch 2 gezeigt, oder Mischungen davon, mit einem Zusatzstoff.

**12.** Zusammensetzung nach Anspruch 11, wobei der Zusatzstoff ein Lösungsmittel ist, d. h. C-IX, Cycloxonon, Aceton, Ethylmethylketon etc., mit dem Emulgator Piperonylbutoxid (5%).

**13.** Zusammensetzung nach Anspruch 11, wobei das Verhältnis des Zusatzstoffes zu den aktiven Inhaltsstoffen im Bereich von 80 bis 95% liegt.

**14.** Zusammensetzung nach Anspruch 11, wobei die Menge, die zum Kontrollieren der fraßhemmenden Aktivität erforderlich ist, im Bereich von 30 bis 60 µg/$cm^2$ liegt.

**15.** Zusammensetzung nach Anspruch 11, wobei die Menge, die zur Verwendung als Insektenwachstumsregulatoraktivität erforderlich ist, im Bereich von 50 bis 100 µg/Insekt für topische Anwendungen oder 5 bis 25 µg für die orale Verfütterung beträgt.

## Revendications

**1.** Composés alkylxanthates à savoir :

(1) le bis(xanthate de tétrahydrofurfuryle) de méthylène,
(2) le n-butylxanthate de m-fluorobenzyle, et
(3) l'isobutylxanthate de m-fluorobenzyle

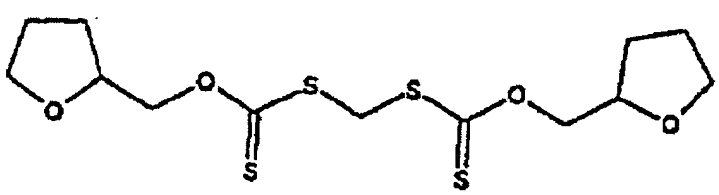

1

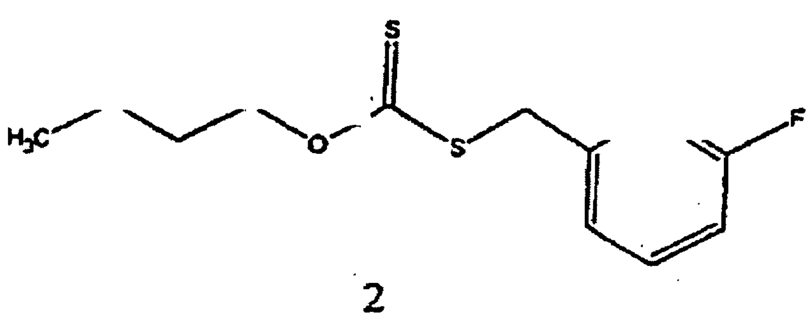

2

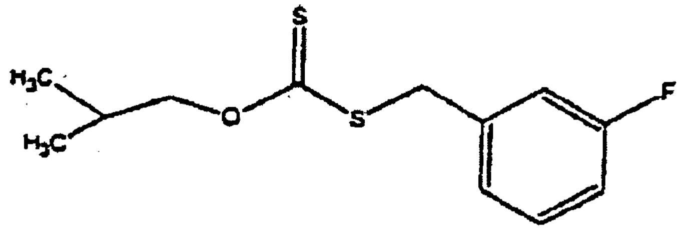

3

2. Emploi d'analogues d'alkylxanthates comme régulateurs de croissance des insectes pour la gestion intégrée des ravageurs (GIR), lesdits analogues étant choisis parmi un groupe constitué du bis(xanthate de tétrahydrofurfuryle) de méthylène, du n-butylxanthate de m-fluorobenzyle, de l'isobutylxanthate de m-fluorobenzyle ou du bis(xanthate de n-butyle) de méthylène, représentés respectivement par les formules 1, 2, 3 et 5.

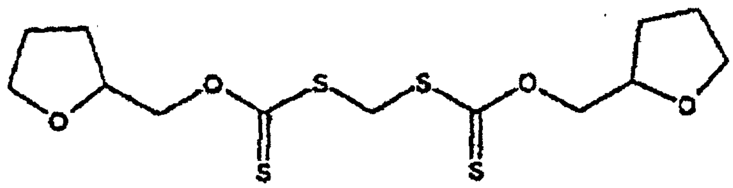

1

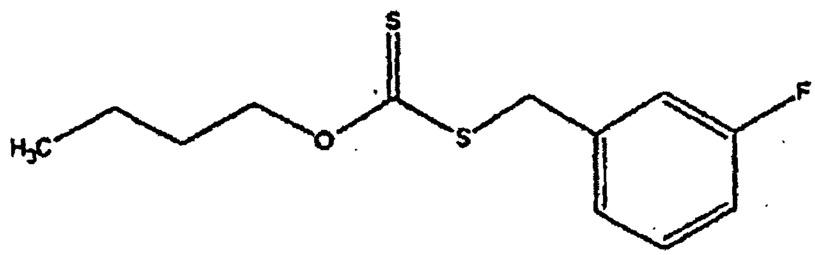

2

2

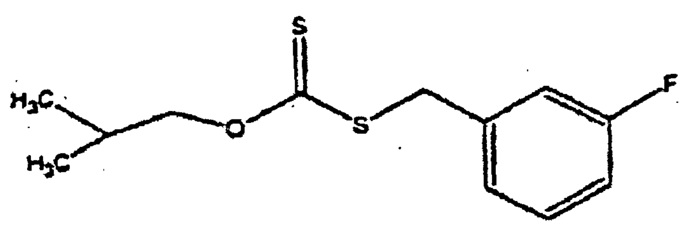

3

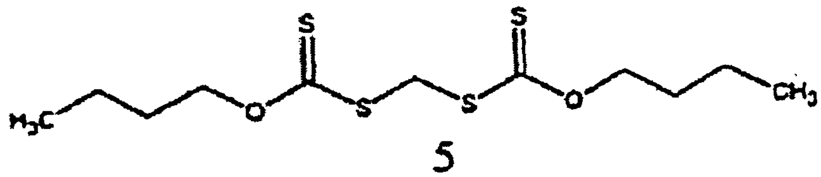

5

3. Emploi tel que revendiqué selon la revendication 2, dans lequel l'alkylxanthate régule la croissance des ravageurs agricoles choisis parmi le groupe constitué de *Spodoptera litura* et d'*Helicover armigera.*

4. Emploi tel que revendiqué selon la revendication 2 dans lequel, la quantité d'alkylxanthates employés comme régulateurs de croissance des insectes s'étend de 50 µg à 100 µg par insecte pour l'application topique sur des larves au stade larvaire III et de 5 à 25 µg pour l'alimentation buccale.

5. Emploi tel que revendiqué selon la revendication 2, dans lequel, le taux de mortalité larvaire se situe dans la plage allant entre 50 à 90 %.

6. Emploi d'analogues d'alkylxanthates comme antiappétants pour la gestion intégrée des ravageurs, lesdits analogues étant choisis parmi le bis(xanthate de tétrahydrofurfuryle) de méthylène, le n-butylxanthate de m-fluorobenzyle, l'isobutylxanthate de m-fluorobenzyle ou le bis(xanthate de n-butyle) de méthylène et représentés par les formules chimiques 1, 2, 3 et 5 comme montré dans la revendication 2.

7. Emploi tel que revendiqué dans la revendication 6 dans lequel, la quantité d'analogues de xanthates d'alkyle employée pour protéger la feuille se situe dans la plage s'étendant entre 15 à 50 microgrammes par $cm^2$ de surface foliaire.

8. Emploi tel que revendiqué dans la revendication 6 dans lequel, le xanthate d'alkyle agit contre les ravageurs polyphages, Spodoptera litura (chenille du tabac) et régule la consommation des feuilles par les ravageurs.

9. Procédé de préparation d'analogues de xanthates d'alkyle tel que défini dans la revendication 2, ledit procédé comprenant les étapes consistant à :

a. refluer du KOH avec un alcool suivi de l'ajout de disulfure de carbone pour obtenir un composé cristallin jaune, à savoir du potassium-O-alkylxanthate,
b. faire réagir le potassium-O-alkylxanthate en présence d'un solvant cétonique avec un dérivé de brome choisi parmi le dibromure de méthylène ou le bromure de m-fluorobenzyle, à filtrer et à éliminer la cétone sous pression réduite pour obtenir les composés alkylxanthates désirés.

10. Procédé tel que revendiqué selon la revendication 9 dans lequel, la réaction est conduite à 0°C.

11. Composition comprenant une quantité efficace d'un analogue alkylxanthate choisi parmi le bis(xanthate de tétrahydrofurfuryle) de méthylène, le n-butylxanthate de m-fluorobenzyle, l'isobutylxanthate de m-fluorobenzyle ou le bis(xanthate de n-butyle) de méthylène des formules 1, 2, 3 et 5 comme montré selon la revendication 2, ou de mélanges de ceux-ci, avec un additif.

12. Composition telle que revendiquée selon la revendication 11 dans laquelle, l'additif est un solvant, c'est-à-dire, C-IX, cycloxonone, acétone, éthyl méthyl cétone etc., avec l'agent émulsifiant butoxyde de pipéronyle (5 %).

13. Composition telle que revendiquée selon la revendication 11 dans laquelle, le rapport de l'additif sur les ingrédients actifs s'étend de 80 à 95 %.

14. Composition telle que revendiquée selon la revendication 11 dans laquelle, la quantité nécessaire pour réguler l'activité antiappétante se situe dans la plage de 30 à 60 µg/$cm^2$.

15. Composition telle que revendiquée selon la revendication 11 dans laquelle, la quantité requise pour avoir une activité régulatrice de la croissance des insectes se situe dans la plage de 50 à 100 µg/insecte pour les applications topiques ou de 5 à 25 µg pour l'alimentation buccale.

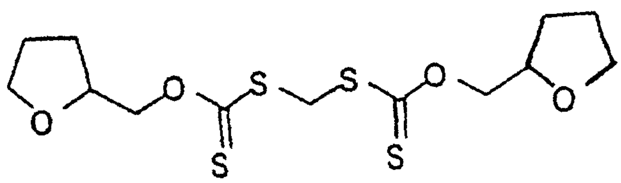

Figure-1

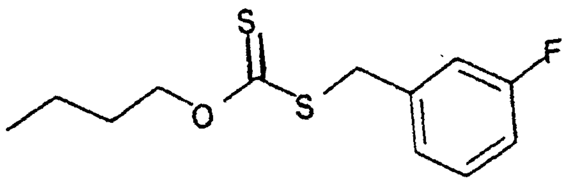

Figure-2

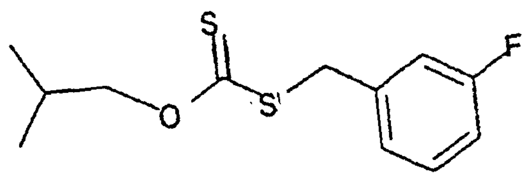

Figure-3

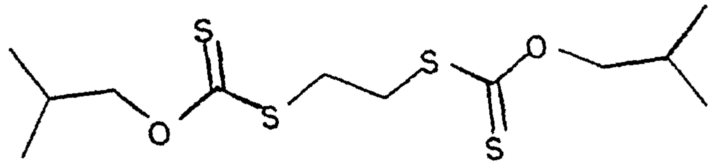

Figure-4

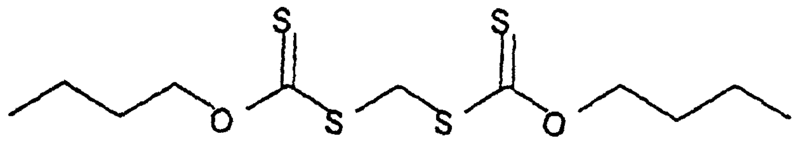

Figure-5

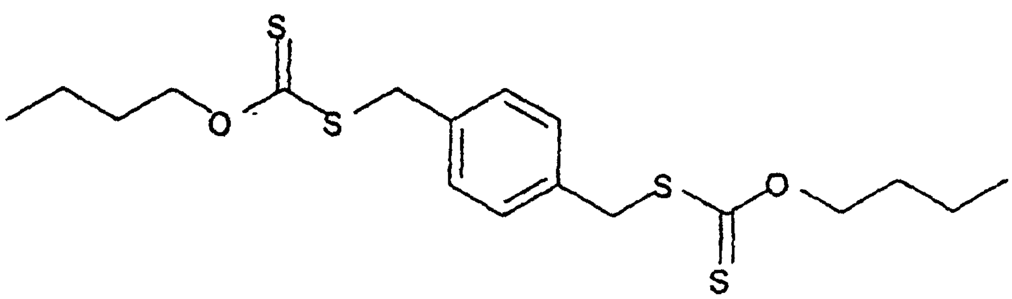

Figure-6

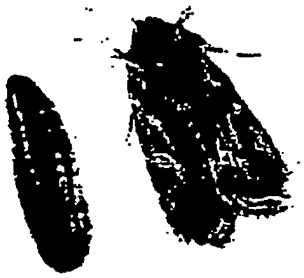

Fig.7(a)

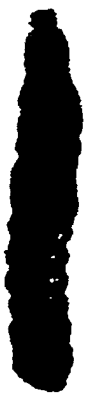

Fig.7(b)

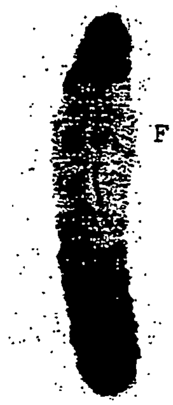

Fig.7(c)

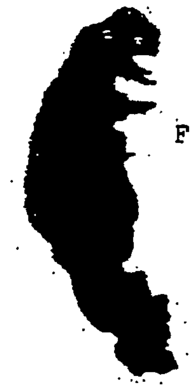

Fig.7(d)

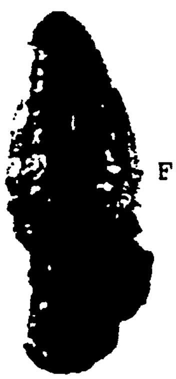

Fig.7(e)

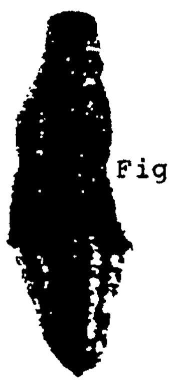

Fig.7(f)

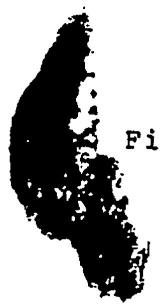

Fig.7(g)

Figure 7

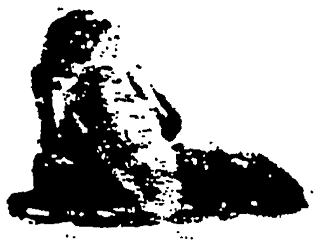

Fig.8(a)

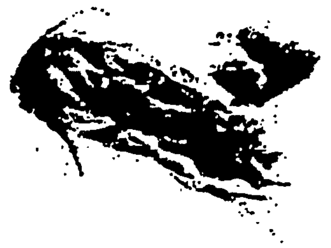

Fig.8(b)

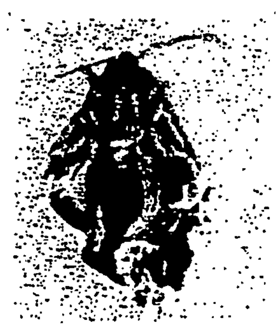

Fig.8(c)

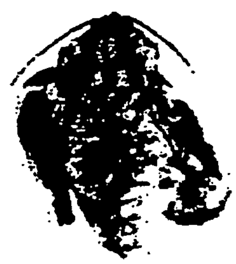

Fig.8(d)

Figure 8